**Europäisches Patentamt**

**European Patent Office**

**Office européen des brevets**

(19)

(11) Veröffentlichungsnummer : **0 033 397**
**B1**

# EUROPÄISCHE PATENTSCHRIFT

(12)

(45) Veröffentlichungstag der Patentschrift :
07.11.84

(51) Int. Cl.³ : **C 12 N 11/04**

(21) Anmeldenummer : **80200089.3**

(22) Anmeldetag : **01.02.80**

(54) **Verfahren zur Herstellung von mechanisch und chemisch stabilen porösen Biokatalysatoren mit hoher enzymatischer Aktivität.**

(43) Veröffentlichungstag der Anmeldung :
**12.08.81 Patentblatt 81/32**

(45) Bekanntmachung des Hinweises auf die Patenterteilung : **07.11.84 Patentblatt 84/45**

(84) Benannte Vertragsstaaten :
**AT BE CH FR GB IT LU NL SE**

(56) Entgegenhaltungen :
J. KLEIN et al. "Procedures for polymer entrapment of whole cells" DECHEMA-Monographs, vol. 84, CHARACTERIZATION OF IMMOBILIZED BIOCATALYSTS, Ed. by K. Buchholz, 274-276, Verlag Chemie-Weinheim-New York, 1979
J. KLEIN et al. "Polymernetzwerke zum Einschluss von Mikroorganismen" Die angewandte Makromolekulare Chemie, 76/77, Nr. 1141, 329-350 (1979)

(73) Patentinhaber : **Klein, Joachim,Prof. Dr.**
**Hühnerkamp 21**
**D-3300 Braunschweig (DE)**

**Wagner, Fritz, Prof. Dr.**
**Hohe Wiese 2**
**D-3300 Braunschweig-Stöckheim (DE)**

(72) Erfinder : **Klein, Joachim, Dr.**
**Hühnerkamp 21**
**D-3300 Braunschweig (DE)**
Erfinder : **Wagner, Fritz, Dr.**
**Hohe Wiese 2**
**D-3300 Braunschweig-Stöckheim (DE)**
Erfinder : **Vorlop, Klaus Dieter**
**Hochstrasse 7**
**D-3300 Braunschweig (DE)**
Erfinder : **Eng, Harald**
**Konrad-Adenauer-Strasse 87**
**D-3320 Salzgitter 1 (DE)**

(74) Vertreter : **Jahn-Held, Wilhelm W., Dr.Dr.-Ing.**
**Dipl.Chem.**
**Schöne Aussicht 8**
**D-3513 Staufenberg 1 (DE)**

## Beschreibung

Biokatalysatoren sind für die direkte Gewinnung von primären und sekundären Stoffwechselprodukten von zunehmender Bedeutung. Als Beispiele aus der Praxis, für welche technologisches Interesse besteht, werden benannt :

Gewinnung von Fructose aus Glukose mittels Glukoseisomerase,

Herstellung von 6-APS aus Penicillin G mittels Penicillin-Acylase,

Herstellung von L-Asparaginsäure aus Ammoniumfumarat mittels E. Coli,

Herstellung von L-Apfelsäure mittels Fumarase mit Brevibakterium ammoniagenes-Zellen.

Unter dem Begriff « Biokatalysator » wird in der technischen Mikrobiologie und im « microbial engineering » ein mittels eines makroskopischen Trägers fixiertes biologisches System aus ganzzelligen Mikroorganismen, oder Enzymen verstanden. In jüngerer Zeit wird dem Einsatz von fixierten Mikroorganismen als Biokatalysator aus Gründen der Herstellungskosten und der Flexibilität des Verfahrens der Herstellung, besonders auch unter dem Gesichtspunkt von Mehrenzymreaktionen, der Vorzug gegeben.

Die Herstellung der meisten Biokatalysatoren erfolgt im wesentlichen durch physikalischen Einschluss des Mikroorganismus in eine Polymermatrix. Zur Herstellung derartiger Matrices wurden nach unterschiedlichen Verfahrensschemen folgende Stoffe verwendet :

Polyacrylamid, Polymethacrylamid, Collagen, Cellulose-Triazetat, Carboxymethylcellulose, Agar, Copolymerisate aus Maleinsäure und Styrol, Carageenan.

Nach dem Stand der Technik weisen die bekannten Verfahren zur Herstellung von Biokatalysatoren mit diesen Stoffen Schwierigkeiten bei ihrer technischen Herstellung auf und es bestehen Nachteile in den Eigenschaften der nach solchen Verfahren erzeugten Biokatalysatoren.

In der Gruppe Alginat-, CMC-, Copolymerisat-Katalysatoren, die in einfacher Weise durch Gelbildung mit mehrwertigen Kationen hergestellt werden, besteht der besondere Nachteil der Inresistenz gegenüber Phosphatpuffer-Lösungen, in denen eine Vielzahl von Reaktionen in der Praxis durchgeführt werden. Bei Verwendung natürlicher Elektrolyte kann ein mikrobieller Befall und damit verbunden, eine Zerstörung der Matrix eintreten.

Dazu kommt bei diesem Katalysatortyp wegen der nicht sehr hohen mechanischen Belastbarkeit die Einschränkung, dass eine Verwendung im Festbettreaktor kaum möglich ist.

Die Herstellung des Cellulosetriacetat-Katalysators nach einem Nassspinnprozess stellt unter Verwendung von stark toxischen Lösungsmitteln wie Toluol oder Methylenchlorid eine Fixierungsmethode dar, die nur für wenige Mikroorganismen in Frage kommt. Durch die Faserform ist der einseitige Einsatz des Katalysators in Festbettreaktoren vorgegeben.

Die Herstellung der Collagen-Katalysatoren ist sehr aufwendig. Der Einsatz ist wegen ihrer Menbranform auf den Spiralreaktor beschränkt. Es kann auf den toxischen Schritt der Härtung mit Glutardialdehyd nicht verzichtet werden.

Polyacrylamid-Katalysatoren sind als Produkte einer Block-Polymerisation als scharfkantige, unregelmässige Granulate beschrieben. Bei Einsatz in Rührreaktoren zeigen diese hohen Abrieb, die Beladung mit Mikroorganismen ist begrenzt. Dies bestätigen die folgenden Offenlegungsschriften :

DE-OS 2 252 815 : 4,3 g E. coli BFM in 120 ml Katalysator,

DE-OS 2 420 102 : 17 g Zellen BFM in 170 ml Katalysator,

DE-OS 2 414 128 : 12 g Zellen BFM in 120 ml Katalysator.

Es haben sich jedoch trotz intensiver Forschung nur wenige Verfahren in die Praxis einführen können. Dies liegt offenbar an folgenden Nachteilen : Die mechanische Stabilität vieler Träger ist zu gering, um diese in grösseren Reaktoren einzusetzen. Die Fixierungsverfahren sind zu aufwendig, sodass der Einsatz solcher Biokatalysatoren nicht wirtschaftlich ist.

Es sind die zu erreichenden Beladungen zu gering, sodass nur eine ungünstige Raum-Zeit-Ausbeute zu erreichen ist.

Eines der bekanntesten Verfahren verwendet den physikalischen Einschluss in ein polymeres Netzwerk.

Beispiele sind : Polyacrylamid/Polymethacrylamid, Collagen, Cellulosetriacetat, ionotrope Gele.

Diese Fixierungsverfahren werden angewendet, da bei diesen relativ geringe Inaktivierungen der Enzyme, Zellfragmente oder der ganzen Zellen auftreten.

Es ist bekannt, dass der Einschluss in ionotrope Gele ein Verfahren darstellt, bei dem im Gegensatz zu den Polyacrylamid- oder Polymethacrylamid-Gelen mit praktisch untoxischen Substanzen gearbeitet wird, wie z. B. mit Alginat, $Ca^{2+}$.

M. Kierstein und C. Bucke berichten in Biotechn. & Bioeng. *19* (1977), S. 387, von dem Einschluss von Enzymen, Zellfragmenten und ganzen Zellen in Ca-Alginat-Gele. Diese Autoren schlagen vor, Fasern durch Eindüsen einer Na-Alginat-Lösung in ein $Ca^{2+}$-Fällungsbad herzustellen.

Diese Fasern weisen jedoch nach Angaben dieser Autoren nur eine mässige Stabilität auf. Ein weiterer Nachteil liegt darin, dass derartige Fasern als Biokatalysatoren lediglich in Festbettreaktoren eingesetzt werden können.

U. Hackel schlägt in seiner Dissertation, Technische Universität Braunschweig, 1976 : « Polymereinschluss von Mikroorganismen-zu Aufbau und Reaktivität von Biokatalysatoren », vor, durch Eindüsen der Polyelektrolytlösung in ein Vernetzerbad kugelförmige ionotrope Gele als Biokatalysatoren herzustellen. Dieser Autor weist auch auf die Verwendbarkeit verschiedener Typen von Polyelektrolyten hin ; dazu gehören auch vollsynthetische Produkte wie Styrol/Maleinsäure-Copolymer.

Diese Vorschläge haben jedoch nicht zu Biokatalysatorperlen mit hoher Druckfestigkeit (pond/Perle) und hoher Beladung an enzymatisch aktiver Substanz in Form von Katalysatorperlen durch Polymereinschluss von Biomasse geführt.

Die Erhöhung der Festigkeit durch Erhöhung des Polymergehaltes im Biokatalysator scheitert daran, dass hochviskose Lösungen nicht mehr zu verarbeiten sind.

Aufgabe der Patentanmeldung ist es, Biokatalysatorperlen mit hoher Druckfestigkeit (pond/Perle) und hoher Beladung an enzymatisch aktiver Substanz (in Gramm enzymatisch aktiver Substanz pro Gramm Katalysatorperlen) in Form von Katalysatorperlen durch Polymereinschluss von Biomasse zur Verfügung zu stellen.

Diese Aufgabe wird durch die in den Ansprüchen 1-14 definierten Gegenstände gelöst.

Ein technischer Effekt des Verfahrens der Erfindung wird durch den folgenden Versuch demonstriert :

Es werden in bekannter Weise Gelperlen durch Vernetzung einer 8 %igen Alginatlösung mit einer 2 %igen $CaCl_2$-Lösung erzeugt.

Die erfindungsgemässe Weiterverarbeitung erfolgt in folgender Weise :

Durch schonende Trocknung mit Luft bei Raumtemperatur über 5 h schrumpfen die Partikel von 3,5 mm auf 1,5 mm, entsprechend 2/5-tel des ursprünglichen Durchmessers. Bei erneuter Äquilibrierung dieser Perlen mit einer 2 %igen $CaCl_2$-Lösung erfolgt eine Rückquellung überraschend nur auf 2 mm.

Nach einem technologischen Einsatz von 6 Wochen bei 65 °C in einer 30 %igen Glucoselösung fand keine weitere Quellung statt.

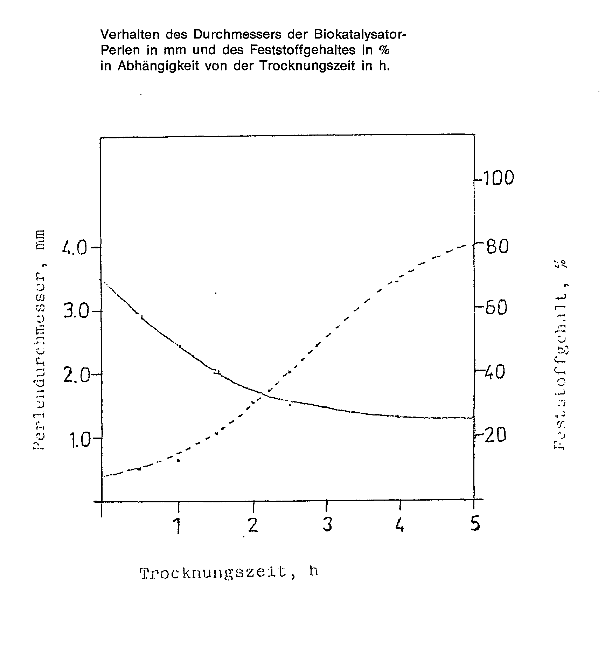

Das Verhalten des Durchmessers des erfindungsgemäss nach Anspruch 1 erhaltenen Biokatalysators (in mm) und des Feststoffgehaltes in % in Abhängigkeit von der Trockenzeit in h zeigt die graphische Darstellung (Figur 1).

Nach diesem Beispiel nimmt der Durchmesser der Biokatalysatorperlen bis zu 2 h Trocknungszeit rasch ab und geht danach bis zu 5 h in eine flache Kurve und damit zu einer langsameren Abnahme des Durchmessers über. Die Zunahme des Feststoffgehaltes zeigt nach 1 und 4 h einen flachen Wendepunkt und verläuft dazwischen mit fast gleichmässiger Steigung. Der Anstieg des Feststoffgehaltes kann als ziemlich regelmässig bezeichnet werden.

Die Erfindung wird durch die folgenden Ausführungsbeispiele erläutert. Dabei beziehen sich die Beispiele 1-3 auf die Ansprüche 1-6 und die Beispiele 4-5 auf die Ansprüche 7-14.

## Beispiel 1

Es wird zunächst die Mischung (A) durch Suspendieren von 150 g Presshefe, die mit Wasser auf 150 ml aufgefüllt wurde, als enzymatisch aktive Substanz ($A_1$) in 450 ml einer 8 gew.-%igen Na-Alginat-Lösung als wässrige Lösung eines Polyelektrolyten hergestellt. Als Fällugsbad (B) mit einer Verbindung, die mehrwertige Ionen enthält, die dem Polyelektrolyten der Mischung (A) entgegengesetzt geladen sind, wird eine 0,2 molare $CaCl_2$-Lösung verwendet.

Es wird dann in Stufe 1 die Mischung (A) mittels einer Kapillare mit einem Durchmesser von 0,4 mm unter Rühren in die Mischung (B) eingetropft. Es entstehen perlförmige Teilchen mit einem Durchmesser von 4 mm. Diese werden unter weiterem Rühren bei einer Verweilzeit von 15 min verfestigt.

In Stufe 2 werden sodann die gebildeten Katalysatorperlen mit der eingeschlossenen, enzymatisch aktiven Substanz abfiltriert und mit physiologischer NaCl-Lösung gewaschen.

Die Katalysatorperlen werden dann in Stufe 3 einer schonenden Trocknung durch 20-stündiges Überleiten von Luft bei einer Temperatur von 30 °C in einem Trockenschrank unterzogen. Hierdurch tritt eine Schrumpfung und Verfestigung der Katalysatorperlen ein. Die relative Schrumpfung beträgt gegenüber dem Zustand vor der Trocknung etwa 2/5-tel.

Es wird danach in Stufe 4 die Nachhärtung der Katalysatorperlen durch Eintragen in das Fällungsbad (C) aus einer $Al_2(SO_4)_3$-Lösung einer Konzentration von 0,1 Mol/l in 30 min durchgeführt.

In Stufe 5 werden die Katalysatorperlen einem Waschprozess mit physiologischer NaCl-Lösung unterworfen und im feuchten Zustand ausgeführt.

Die Katalysatorperlen dieses Beispieles besitzen nach der Mengenbilanz eine Beladung an enzymatisch wirksamer Substanz von 0,88 g Presshefe pro g Biokatalysator.

Im Druckfestigkeitsversuch (siehe Beispiel 3) ergibt sich eine Belastbarkeit im Bruchpunkt von 750 p/Perle.

## Beispiel 2

Es wird zunächst die Mischung (A) hergestellt. Hierzu werden 100 g E. coli ATCC 11105 (in Form der zentrifugenfeuchten Masse) in Wasser auf insgesamt 150 ml aufgefüllt und in 450 ml einer 8 gew.-%igen wässrigen Na-Alginat-Lösung als Polyelektrolyt suspendiert. Im übrigen wird nach Beispiel 1 verfahren.

Aus 600 ml Ausgangslösung (A) werden 165 ml Biokatalysatorperlen erhalten. Daraus errechnet sich eine Beladung mit Biofeuchtmasse (BFM) von 0,6 g BFM/ml Biokatalysator. Die Druckfestigkeit liegt bei 800 p/Perle.

Beispiel 3

Es wird zunächst die Mischung (A) durch Lösen von 100 mg Amyloglucosidase ($A_1$) in 10 ml einer 8 %igen, wässrigen Na-Alginat-Lösung hergestellt. Als Fällungsbad (B) mit einer Verbindung, die mehrwertige Ionen enthält, die dem Polyelektrolyt der Mischung (A) entgegengesetzt geladen sind, wird eine 0,5 molare $CaCl_2$-Lösung verwendet.

In Stufe 1 wird dann die Mischung (A) aus einer Kapillaren mit einem Durchmesser von 0,4 mm in die Mischung (B) unter Rühren eingetropft, wobei perlförmige Teilchen mit einem Durchmesser von 4 mm entstehen. Diese werden unter weiterem Rühren bei einer Verweilzeit von 15 min verfestigt.

In Stufe 2 werden die gebildeten Katalysatorperlen mit der eingeschlossenen enzymatisch aktiven Substanz abfiltriert und mit physiologischer NaCl-Lösung gewaschen.

Es werden dann die Katalysatorperlen in Stufe 3 einer schonenden Trocknung durch 20-stündiges Überleiten von Luft bei einer Temperatur von 30 °C getrocknet. Es tritt bei dieser Massnahme die Schrumpfung und Verfestigung der Katalysatorperlen ein. Bei einem Durchmesser von nunmehr 1,5 mm beträgt die relative Schrumpfung etwa 2/5-tel.

Es wird danach in Stufe 4 die Nachhärtung der Katalysatorperlen durch Eintragen in das Fällungsbad (C), bestehend aus einer 0,1 molaren $Al_2(SO_4)_3$-Lösung, über einen Zeitraum von 30 min durchgeführt.

Es werden danach in Stufe 5 die Katalysatorperlen einem Waschprozess mit physiologischer NaCl-Lösung unterworfen und in feuchtem Zustand ausgeführt.

Die Biokatalysatorperlen besitzen im Endzustand einen Durchmesser von 1,8 mm, woraus sich nach Massgabe der Mengenbilanz eine Beladung von 32 mg Enzym/ml Biokatalysator ergibt.

Der Druckfestigkeitsversuch ergibt eine Festigkeit von 780 p/Perle.

Es wird nachstehend die Messmethode zur Bestimmung der Druckfestigkeit beschrieben.

· Der mechanische Teil der Testanordnung besteht aus einem fest eingespannten Probetisch, einem darüber angeordnetem Stempel, der starr mit einem Kraftaufnehmer (Fa. Hottinger Baldwin Messtechnik, Darmstadt ; Kraftaufnehmer U 1/l kpond) verbunden ist und einer Antriebseinheit aus Motor und Getriebe. Das Signal des Kraftaufnehmers wird in einem Messverstärker, Typ KWS 3 072 (Fa. HBM, Darmstadt) verstärkt und auf einen Schreiber gegeben.

Zur Messung der Belastungsfähigkeit von Katalysatorkugeln wird jeweils eine Kugel auf den Probetisch gelegt. Von oben drückt dann der Stempel mit einer Vorschubgeschwindigkeit von 1,45 mm/min auf das Katalysatorkorn.

Die auf die Kugel wirkende Kraft wird über Stempel, Kraftaufnehmer und Verstärker direkt als Messgrösse durch den Schreiber in einem Kraft-Zeit-Diagramm aufgezeichnet.

Aus diesem Diagramm lassen sich reproduzierbare Aussagen über die Bruchfestigkeit dadurch machen, dass sich der Bruchvorgang durch eine zahnartige Diskontinuität im Kraft-Zeit-Diagramm eindeutig markieren lässt.

Die Fehlergrenze der Kraftmessung selbst liegt unter 1 %. Bei der Vermessung mehrerer Perlen einer Herstellungscharge ergeben sich naturgemäss grössere Schwankungen, die jedoch ± 5 % nicht überschreiten.

Durch dieses Messverfahren wird gleichzeitig die Druckfestigkeit in der Dimension « Pond/Perle » als eine an die Geometrie der jeweiligen Perle gebundene Grösse definiert.

Die Krafteinheit « pond » kann in die SI-Einheiten « Newton » wie folgt umgerechnet werden :
1 000 pond = 9 806 Newton ≈ 10 Newton.

Nach dieser Methode zeigt sich, dass nicht gehärtete Ca-Alginat-Perlen nach dem Stand der Technik mit einem Durchmesser von 3,5 mm bereits bei einer Druckkraft von 10 bis 150 p zerstört werden. Dagegen werden nach dem Verfahren der Erfindung erzeugte Biokatalysatorperlen mit einem Durchmesser von 2 mm erst bei Anwendung einer Druckkraft von über 600 bis 1 000 p/Perle zerstört.

Die nach dem Verfahren der Erfindung (Anspruch 1-6) hergestellten Biokatalysatorperlen weisen aufgrund der erreichbaren Volumenverminderung eine Anreicherung der Biomasse auf, die nach dem Stand der Technik nicht erreichbar ist.

M. Kierstein und C. Bucke (l.c.) erreichen einen Wert von 0,25 g $BFM/cm^3$ Kat.
(BFM = Biofeuchtmasse. Kat. = Katalysator).

In den Deutschen Offenlegungsschriften 2 252 815, 2 420 102, 2 414 128 werden bei der Fixierung in Polyacrylamidgelen nur Beladungen von 0,04 bis 0,1 g $BFM/cm^3$ Kat. erreicht.

Dagegen weisen nach dem Verfahren der Erfindung hergestellte Biokatalysatorperlen eine Beladung von 0,88 g BFM/g Katalysatorperlen auf.

Die nach dem Verfahren der Erfindung hergestellten Katalysatorperlen zeigen jedoch auch eine hohe Porosität, wie aus Aufnahmen mit dem Rasterelektronenmikroskop (REM) zu ersehen ist.

Die hohe Beladung an enzymatischer Aktivität wird beispielsweise bei der Umsetzung von Penicillin G mit erfindungsgemäss immobilisierten E. coli-Zellen durch eine relative Aktivität von 61 % und eine absolute Aktivität von 4 400 U (Units)/liter Katalysator bestätigt.

4

(Biokatalysatorperlen mit 2,5 mm Durchmesser; Beladung 0,5 g BFM/cm$^3$; Temperatur 37 °C; Konzentration an Penicillin G Na-Salz von 5 %.)

## Beispiel 4

30 g E. coli-Zellen ATCC 11105 werden in Form der abzentrifugierten, fliessfähigen Biofeuchtmasse (BFM) (Komponente ($A_1$)) mit 10 g des Epoxiharzes « Epikote Dx-255 » (Deutsche Shell AG, Frankfurt) als Harzkomponente ($A_2$) vermischt.

In Stufe 1 werden dann in dem Gemisch 20 g einer 25 %igen wässrigen Lösung von « Casamide CA 360 » (Akzo-Chemie, Düren) als Polyaminoamid-Härter (Komponente ($B_1$)) durch Rühren gut verteilt, wodurch die Polykondensation des Epoxiharzes eingeleitet wird. Dieses System ($A_2$) + ($B_1$) + ($A_1$) wird nun mit 20 ml einer 8 gew.-%igen wässrigen Na-Alginat-Lösung (« Mannucol LD » der Fa. Alginate Ind., Hamburg) als Lösung (D) gut vermischt. Das Gemisch wird danach in ein Fällungsbad (eine 2 gew.-%ige CaCl$_2$-Lösung (Komponente (E)) mittels einer Kapillaren mit dem Durchmesser 0,4 mm durch Anlegen von Überdruck eingedüst. Dabei bilden sich perlförmige Teilchen mit einem Durchmesser von 3-4 mm. Nach 20-minütigem Rühren werden die Teilchen als nunmehr stabile Partikel aus der CaCl$_2$-Lösung entfernt.

Diese Partikel werden in Stufe 2 gewaschen.

An diesen Prozess schliesst sich in Stufe 3 eine schonende Trocknung durch Überleiten von Luft bei 28 °C über einen Zeitraum von 24 Stunden an. Dabei schrumpfen die Perlen auf etwa 2/5-tel ihres ursprünglichen Durchmessers.

Die trockenen bezüglich der Komponenten ($A_2$) und ($B_1$) in Stufe 4 ausgehärteten Perlen werden in Stufe 5 für 40 Minuten in einer 0,1 molaren Phosphat-Pufferlösung unter Rühren gewaschen, wobei das Alginat als Perlelektrolytkomponente (D) aus den Kugeln herausgelöst wird und die Perlen auf einen Durchmesser von 3 mm rückquellen.

Der so erhaltene poröse Biokatalysator weist eine hohe Beladung an Zellmasse bei einer hohen Aktivität mit hoher mechanischer Stabilität auf, wie die folgenden Daten zeigen:

| Katalysator-Typ | Beladung E. coli g BFM pro g Katalysator | Beladung Vol.-% | Relative Aktivität % (*) | Absolute Aktivität Kat/l Katalysator |
|---|---|---|---|---|
| Unregelmässige Partikel aus Blockkondensation | | | | |
| ∅ 100 μm | 1,24 | 70 | 40 | 80 |
| ∅ 3-4 mm | 1,24 | 70 | 11 | 16,4 |
| Kugel-Perlen nach dem Verfahren der Erfindung ∅ 3 mm | 1,13 | 67 | 28 | 38 |

(*) Aktivität der fixierten E. coli-Zellen, bezogen auf die Aktivität der gleichen Zellmenge in freier Suspension.

Bei einer relativen Aktivität von 28 % (fixierte Zellen pro gleicher Zahl freier Zellen) liegt die absolute Aktivität (Penicillin G-Acylase, 37 °C, $p_H$ = 7,8) bei 38 μ Kat./l Katalysator.

Die Druckfestigkeit der Perlen beträgt 651 p/Perle.

## Beispiel 5

35 g Presshefe als Komponente ($A_1$) werden in 10 g des Epoxiharzes (« Epikote Dx-255 », Deutsche Shell AG, Frankfurt) als Harzkomponente ($A_2$) eingerührt.

Darin werden in Stufe 1 20 g einer 25 Gew.-%igen wässrigen Lösung von « Casamide CA 360 » (Akzo Chemie, Düren) als Polyaminoamid-Härter-Komponente ($B_1$) durch Rühren gut verteilt, wodurch die Polykondensation des Epoxiharzes eingeleitet wird. Dieses System ($A_2$) + ($B_1$) + ($A_1$) wird nun mit 35 ml einer 8 Gew.-%igen Na-Alginat-Lösung (« Mannucol LD »; Fa. Alginat Ind., Hamburg) als Lösung (D) gut vermischt und in eine 1 gew.-%ige CaCl$_2$-Lösung als Komponente (E) aus einer Kapillare mit dem Durchmesser von 0,4 mm durch Anlegen von Überdruck eingedüst. Dabei bilden sich perlförmige Teilchen mit einem Durchmesser von 3-4 mm.

Im übrigen wird nach Beispiel 4 verfahren. Die Biokatalysatorperlen haben schliesslich einen Durchmesser von 3 mm. Die Beladung beträgt 1,18 g Biofeuchtmasse pro g Biokatalysator. Die absolute Aktivität, gemessen am Abbau von Glucose zu Äthanol, beträgt 0,18 g Glucose/ml Katalysator.h. Die Druckfestigkeit beträgt 650 p/Perle Biokatalysator.

Nach dem Verfahren der Erfindung (Ansprüche 7-14) wird der stoffliche Aufbau der Polymermatrix eines Biokatalysators unter Benutzung der Polykondensation einer mehrfunktionellen, wasser-emulgierbaren Epoxi-Praepolymer-Komponente (A₂) mit einer mehrfunktionellen Härtungskomponente (B₁), wie Polyaminoamid, vollzogen.

Der technische Vorteil liegt darin, dass der Polymeraufbau bei geeigneter Auswahl der Reaktionskomponenten bei Raumtemperatur ohne Bildung von Nebenprodukten abläuft. Das Polymernetzwerk weist eine hohe mechanische und chemische Stabilität auf. Die Behandlung mit Salzlösung sowie extreme pH-Werte im sauren und alkalischen Bereich führen zu keiner Beeinflussung der materiellen Netzwerkeigenschaften.

Die in dem Verfahren der Erfindung in wässrigem Medium unter Ausschluss schädlicher Lösungsmittel verwendeten Epoxi-Praepolymeren führen zu praktisch untoxischen Biokatalysatorperlen.

Das direkte Einbringen der feuchten Biomasse (A₁) in die viskose Epoxiharz-Komponente (A₂) führt zur Umhüllung der Zellen mit der Komponente (A₂), wobei das untoxische Harz zusätzlich die Funktion eines Schutzkolloides während der Fixierung übernimmt.

Die besondere Eignung der Epoxide zum Einschluss ganzer Zellen oder Enzymen ergibt sich bereits bei der einfachen Blockkondensation von (A₂) + (A₁) mit (B₁). Durch den hohen Anteil an Biomasse (A₁) im Verhältnis zur eingesetzten Menge an Harz- und Härtungs-Komponente (A₂) + (B₁) wird, wie Untersuchungen mit dem Rasterelektronenmikroskop bestätigen, erreicht, dass ein durch Blockpolymerisation synthetisiertes Epoxi-Polymernetzwerk Porosität aufweist.

Die mit der Blockpolymerisation unter Wasseraustritt einhergehende Schrumpfung des Trägermaterials der nach dem Verfahren der Erfindung verwendeten Stoffe (A₂) und (B₁) führt zu hohen Beladungen an Biomasse (A₁) mit hoher enzymatischer Aktivität.

Es wird jedoch bei dieser Arbeitsweise mit der für technische Prozesse notwendigen Körnung von etwa 3-4 mm nur eine geringe enzymatische Aktivität von etwa 1/5-tel der ursprünglichen Aktivität erreicht. Dieses Ergebnis ist auf eine Diffusionshemmung zurückzuführen. Durch die hohe Beladung mit Biomasse (A₁) können die Substratmoleküle nicht alle Zellen erreichen.

Diese Feststellung wird durch die folgende Untersuchung bestätigt. Wenn ein die Zellen enthaltender Epoxiblock in einer Polymermühle in scharfkantige, unregelmässig geformte Katalysatorpartikel von 50 bis 100 Mikron zerkleinert wird, so zeigen diese Partikel bereits eine höhere enzymatische Aktivität. Dies spricht für eine relativ milde Fixierungsart. Damit wird auch das REM-Ergebnis (REM = Raster-Elektronenmikroskop) bezüglich der Porosität bestätigt.

Mit zunehmender Grösse der Körnung derartiger, gemahlener Katalysatorpartikel ist jedoch eine Abnahme der Aktivität verbunden. Derartige Produkte sind also für den Einsatz in der Technik nicht geeignet.

Dagegen besitzen die nach dem Verfahren der Erfindung hergestellten Katalysatorperlen auch in der technisch notwendigen Korngrösse eine hohe enzymatische Aktivität.

Die lange Zeit bis etwa 30 h zur Trocknung, vorzugsweise bei Raumtemperatur (Stufe 3) mit Aushärtung der Katalysatorperlen (Stufe 4), die auch als lange « Topfzeit » der Ausgangsstoffe bezeichnet wird, bietet den Vorteil der bei technischen Fixierungsverfahren erwünschten Zeitreserven.

Nachteilig bei der Arbeitsweise nach dem Stand der Technik ist der grosse Aufwand an Vermahlungsenergie. Dazu kommt ein erheblicher Abrieb der unregelmässig getrockneten, spröden Partikel, sowie die Reaktionshemmung durch Diffusionslimitierung. Diese Nachteile werden durch das Verfahren der Erfindung vermieden.

Nach dem Verfahren der Erfindung wird durch die Kombination (A₂) + (B₁) mit der ionotropen Gelbildung (D) + (A₁) ein perlförmiger, poröser Biokatalysator erzeugt, der gegenüber den Biokatalysatoren nach dem Stand der Technik eine hohe mechanische und chemische Stabilität, sowie eine hohe Beladung an enzymatischer Biomasse aufweist, die auch im technisch wichtigen Kornbereich von etwa 2 bis 5 mm eine hohe enzymatische Aktivität besitzt und durch seine perlförmige Formgebung und seine Festigkeit in den bekannten Reaktorsystemen einsetzbar ist.

Eine bevorzugte Ausführungsform (Anspruch 12) besteht in der Beimengung eines Polyelektrolyten (D), wie Na-Alginat, zur Epoxi-Härter-Biomasse (A₂) + (B₁) + (A₁) durch periodisches Eindüsen in ein Fällungsbad (E) aus wässriger CaCl₂-Lösung, wodurch die unmittelbare ionische Vernetzung des Alginats an der Tropfenoberfläche und damit die Bildung des perlförmigen Katalysators ermöglicht wird. Dabei beginnt sich gleichzeitig die Polykondensation des Harz-Härter-Systems (A₂) + (B₁) innerhalb der Ca-Alginathülle zu vollziehen.

Nach wenigen Minuten sind die Tropfen soweit zu Perlen verfestigt, dass diese abgetrennt und gewaschen werden können und der schonenden Trocknung durch Überleiten von Luft und der Aushärtung unterworfen werden können.

Nach Beendigung des Trocknungsvorganges von etwa 20 h bei Raumtemperatur wird das in den Katalysatorperlen gleichmässig verteilte Alginat, z. B. mittels Phosphatpuffer-Lösung herausgewaschen. Es werden durch diese Massnahmen durchgehende Kapillarwege gebildet und es entsteht der poröse, perlförmige Biokatalysator, der im wesentlichen aus Epoxid (A₂) + (B₁) und der Biomasse (A₁) besteht. Die durch den Einschluss des Alginats in Stufe 1 und durch das Herauslösen in Stufe 5 zusätzlich gewonnene Porosität, optisch erkennbar an den REM-Aufnahmen, ist an einem Quellen der Katalysatorperln beim Herauswaschen des Alginats um 30 % gegenüber den getrocknetes Alginat enthaltenden Perlen festzustellen.

Durch die Strukturveränderung des Epoxinetzwerkes, hervorgerufen durch die Polykondensation von $(A_2) + (B_1)$ in einer Polyelektrolytmatrix (D) + (E), ist das makroskopische Stoffverhalten der Trägermatrix von einem spröden Verhalten bei den Blockkondensations-Partikeln zu einem elastischem Verhalten bei den Katalysatorperlen übergegangen. Dieser technische Vorteil ist für den universellen Einsatz der Katalysatorperlen von erheblicher Bedeutung.

Die folgende Tabelle zeigt die technische Überlegenheit der Katalysatorperlen gemäss der Erfindung gegenüber solchen nach dem Stand der Technik in Bezug auf die Druckfestigkeit, ausgedrückt in pond/Perle.

| Trägersystem (1) bis (4) siehe Fussnote | Druckfestigkeit pond/Perle |
|---|---|
| Polyacrylamid (1) | kleiner 10 |
| Polymethacrylamid (1) | 30 bis 80 |
| Copoly-Maleinsäure-Styrol (2) | 200 bis 400 |
| Epoxi-Block (3) | grösser 1000 |
| Epoxi-Perlen gemäss der Erfindung | 650 bis 895 |

Ein Abrieb der erfindungsgemäss hergestellten Katalysatorperlen ist unter extremen Bedingungen im batsch-weisen Rührkesselbetrieb nicht feststellbar.

1) P. Schara, Dissertation Technische Universität Braunschweig, 1977.
2) U. Hackel, Dissertation Technische Universität Braunschweig.
3) Nicht veröffentlichte Untersuchung mit Epoxiblock nach dem Stand der Technik.

In der folgenden Tabelle wird vergleichsweise die Druckfestigkeit von handelsüblichen Ionen-Austauscher-Harzen in pond/Perle nach der gleichen Messmethode angegeben:

Typ « LEWATIT », Handelsprodukte der Bayer AG, Leverkusen.
Material : Styrol-Divinylbenzol (DVB).
Der « DVB »-Gehalt gibt den Vernetzungsgrad an.

| Makroporös : | Typenbezeichnung | DVB % | Druckfestigkeit pond/Perle |
|---|---|---|---|
| Lewatit | SPC 108/H | 8 | 380 |
| Lewatit | SP 112 | 12 | 710 |
| Gelförmig : | | | |
| Lewatit | SC 104/H | 4 | 50 |

Dieser Vergleich bestätigt die hohe Druckfestigkeit der Katalysatorperlen gemäss der Erfindung gegenüber bekannten Ionenaustauscher-Harzen.

Die enzymatische Langzeit-Stabilität eines Biokatalysators ist eine wichtige Voraussetzung für den technischen Einsatz unter wirtschaftlichen Bedingungen. Ein nach dem Verfahren der Erfindung hergestellter, perlförmiger Biokatalysator mit in Epoxid-Perlen fixierten E. coli-Zellen, der bei 9 °C in 0,9 %iger NaCl-Lösung gelagert ist, besitzt nach 120 Tagen noch etwa 21 % der ursprünglichen Aktivität. Unter gleichen Bedingungen gelagerte freie Zellen sind bereits nach 3-4 Tagen unbrauchbar.

Ein reaktionskinetischer Langzeittest wurde in einem Wirbelbettreaktor mit Rührkesselcharakteristik durchgeführt. Dazu wurden frisch hergestellte Katalysatorperlen verwendet. Es wird eine Serie von sich wiederholenden « Batsch-reaction-runs » durchgeführt. 1 l einer 0,5 %igen Penicillin G-Lösung wird kontinuierlich zirkulierend durch den die Katalysatorperlen enthaltenden Wirbelbettreaktor gepumpt. Temperatur 37 °C ; pH-Wert = 7,8. Die Reaktionslösung wird alle 24 Stunden vollständig gegen frische 0,5 %ige Penicillin G-Lösung ausgetauscht.

Die enzymatische Aktivität des Biokatalysators bleibt innerhalb von 30 Tagen praktisch konstant.

Der vergleichsweise mit einem bekannten Polyacrylamid-Biokatalysator mit fixierten E. coli-Zellen durchgeführte Langzeittest hat eine Halbwertszeit von 17 Tagen bei 40 °C.

Dazu wird auf « Continous Production of 6-Aminopenicilanic Acid from Penicillin by Immobilized Microbial Cells ». Sato, Tosa, Chibata in European J. Appl. Microbiol., 2, Seite 153-160 (1976) verwiesen.

Die Perlform des erfindungsgemäss hergestellten Biokatalysators gestattet mit sehr guter Raum-Zeit-Ausbeute und Langzeitstabilität den universellen Einsatz in den verschiedensten Reaktortypen. Es wird auch insoweit die Überlegenheit des erfindungsgemäss hergestellten Biokatalysators gegenüber solchen nach dem Stand der Technik nachgewiesen.

Das Verfahren der Erfindung bietet weiter den Vorteil einer grossen Breite in der Variation der

**0 033 397**

Auswahl der Polymerkomponente (A$_2$) und des Vernetzers (B$_1$). Es kann somit das ionotrope Gel den physiologischen Eigenschaften der Biomasse angepasst werden. Die Breite der Variation ergibt sich auch in der Möglichkeit des Einsatzes ganzer Zellen und Enzymen als Biomasse. Es besteht dabei der Vorteil einer hohen Standzeit des erfindungsgemäss hergestellten Biokatalysators.

Ein weiterer technischer und wirtschaftlicher Vorteil des Verfahrens der Erfindung liegt auch darin, dass die Vernetzungsreaktion zur Bildung ionotroper Gele reversibel ist.

Es lässt sich die Polymerkomponente des Biokatalysators nach dessen Verwendung in technischen Prozessen durch Auflösen des Netzwerkes wiedergewinnen.

**Ansprüche**

1. Verfahren zur Herstellung von Biokatalysatoren mit hoher Druckfestigkeit (Pond/Perle) und mit hoher Beladung an enzymatisch aktiver Substanz (in Gramm enzymatisch aktiver Substanz pro Gramm Biokatalysator) in Form von Katalysatorperlen durch Polymereinschluss von Biomasse, dadurch gekennzeichnet, dass die an sich bekannten Mischungen:

A) durch Suspendieren oder Lösen einer enzymatisch aktiven Substanz (A$_1$) aus vermehrungsfähigen ganzen Zellen oder Enzymen in einer 0,5 bis 15 gew.-%igen wässrigen Lösung eines Polyelektrolyten und

B) als Fällungsbad durch Lösen einer Verbindung, die mehrwertige, dem Polyelektrolyten der Mischung (A) entgegengesetzt geladene, Ionen enthält, in Wasser zu einer Konzentration der Verbindung von 0,5 bis 35 Gew.-%, hergestellt werden, dass danach in Stufe 1 in an sich bekannter Weise die Mischung (A) durch Eindüsen in die Mischung (B) in perlförmige Teilchen mit einer bestimmten Korngrösse von 0,5 bis 5 mm übergeführt wird, und diese Teilchen unter Rühren an ihrer Oberfläche innerhalb einer Verweilzeit von 5 min bis 4 h verfestigt werden, dass danach in Stufe 2 in an sich bekannter Weise die gebildeten Katalysatorperlen mit der eingeschlossenen, enzymatisch aktiven Substanz abfiltriert, mit physiologischer Salzlösung gewaschen und in Stufe 3 einer schonenden Trocknung durch Überleiten von Luft bei einer Temperatur bis 80 °C über einen Zeitraum bis 48 h derart unterworfen werden, dass die Katalysatorperlen auf eine relative Schrumpfung von 4/5-tel bis 1/5-tel des Zustandes vor der Trocknung eingestellt und verfestigt werden, dass danach in Stufe 4 die Katalysatorperlen zur Nachhärtung in das gleiche Fällungsbad (B) aus Stufe 1 eingetragen werden, wodurch bei einer gegenüber dem ursprünglichen Zustand begrenzten Rückquellung eine Verfestigung der Katalysatorperlen durch Nach- bzw. Umvernetzung erfolgt, und dass in Stufe 5 die Katalysatorperlen einem Waschprozess unterworfen und im feuchten Zustand ausgeführt werden.

2. Verfahren nach Anspruch 1, dadurch gekennzeichnet, dass die enzymatisch aktive Substanz (A$_1$) in der Lösung (A) ein Enzym ist und in einer Konzentration bis zu 0,5 g/ml der Lösung (A) vorhanden ist, dass in Stufe 1 die erhaltenen perlförmigen Teilchen durch langsames, gleichmässiges Rühren innerhalb einer Verweilzeit von 0,25 bis 5 h verfestigt werden, und dass in Stufe 4 als Fällungsbad (B) das Fällungsbad (B) aus Stufe 1 oder ein Fällungsbad (C) aus einer 0,5 bis 5 gew.-%igen Lösung eines mehrwertigen Elektrolyten eingesetzt wird.

3. Verfahren nach den Ansprüchen 1 und 2, dadurch gekennzeichnet, dass in Mischung (A) als Polyelektrolyt ein natürlicher Polyelektrolyt, z. B. Alginat oder Pektinat im Molekulargewichtsbereich von 20 000 bis 200 000, oder ein natürliches Polymer nach chemischer Modifizierung, z. B. Carboxymethylzellulose eingesetzt wird.

4. Verfahren nach den Ansprüchen 1 bis 3, dadurch gekennzeichnet, dass bei Verwendung von anionischen Polyelektrolyten in Mischung (A) in der Mischung (B) als ionische Verbindung niedermolekulare Salze mit den zum anionischen Polyelektrolyten in (A) entgegesetzt geladenen mehrwertigen Ionen, z. B. CaCl$_2$, Al$_2$(SO$_4$)$_3$, Fe(NO$_3$)$_3$ oder deren Mischungen, in Konzentrationen von 0,05 bis 1 Mol/l eingesetzt werden.

5. Verfahren nach den Ansprüchen 1 bis 4, dadurch gekennzeichnet, dass die Mischung (A) durch Suspendieren der ganzen vermehrungsfähigen Zellen in einer wässrigen 2 bis 10 gew.-%igen Na-Alginatlösung hergestellt wird, und in Stufe 1 als Fällungsbad (B) eine 0,05 bis 1 molare CaCl$_2$-Lösung und in der Stufe 4 als Fällungsbad (C) eine 0,05 bis 1 molare Al$_2$(SO$_4$)$_3$-Lösung, verwendet wird, oder dass die Mischung (A) durch Suspendieren oder Lösen eines isolierten Enzyms oder Enzym-Komplexes in einer 2 bis 10 gew.-%igen Na-Alginatlösung hergestellt wird, und in Stufe 1 und 4 als Fällungsbad eine 0,05 bis 1 molare Fe(NO$_3$)$_3$-Lösung verwendet wird.

6. Verfahren nach den Ansprüchen 1 bis 5, dadurch gekennzeichnet, dass in Stufe 1 die Mischung (A) periodisch in die Mischung (B) eingedüst wird.

7. Verfahren nach dem Oberbegriff des Anspruches 1, dadurch gekennzeichnet, dass eine feuchte, enzymatisch aktive Biomasse (A$_1$) aus vermehrungsfähigen ganzen Zellen oder Enzymen mit einer Harzkomponente (A$_2$) aus einer praktisch reinen, mehrfunktionellen Epoxi-Präpolymer-Komponente im Gewichtsverhältnis (A$_2$) zu (A$_1$) wie 0,5 : 1 bis 5 : 1, vermischt wird, dass danach in Stufe 1 eine wässrige Lösung einer mehrfunktionellen Härtungskomponente (B$_1$) mit der erhaltenen Mischung (M) im Gewichtsverhältnis (B$_1$) : (M), wobei (M) = (A$_2$) + (A$_1$), wie 0,2 : 1 bis 0,8 : 1, vermischt und durch Rühren zur Einleitung der Polykondensation praktisch homogen verteilt wird, dass dieses System mit einer wässrigen Lösung eines Polyelektrolyten (D) im Gewichtsverhältnis 1 : 0,6 bis 1 : 2,8 vermischt wird, dass danach die Mischung in eine im Überschuss vorgelegte wässrige Lösung eines niedermolekularen Elektrolyten mit mehrwertigen Ionen (E) unter Entstehung perlförmiger Teilchen einer bestimmten Körngrösse von 0,5 bis 5 mm eingedüst wird und dass diese Teilchen unter Rühren in 5 bis 50 min. zu äusserlich verfestigten Teilchen gehärtet werden, dass sie in Stufe 2 einem Waschprozess unterworfen werden, dass sie danach in Stufe 3 durch Kontakt mit Luft von einer Temperatur von bis zu 80 °C bis 30 h lang schonend derart getrocknet werden, dass die Katalysatorperlen auf eine relative Schrumpfung von 4/5-tel bis 1/5-tel des Zustandes vor der Trocknung eingestellt und verfestigt werden, dass sie in Stufe 4 ausgehärtet werden, und dass in Stufe 5 die Polyelektrolyte aus den perlförmigen Teilchen durch einen Waschprozess mit einer zur Lösung (E) konkurrierenden, ionischen Lösung einer Konzentration von 0,05 bis 5 m/l herausgelöst, und die perlförmigen Teilchen im feuchten Zustand abgetrennt und ausgeführt werden.

8. Verfahren nach Anspruch 7, dadurch gekennzeichnet, dass als Harzkomponente ($A_2$) ein in Wasser emulgierbares, niedrig viskoses Epoxiharz, oder eine modifizierte Bisphenol A/Epichlorhydrin-Epoxiverbindung, oder ein am Anmeldetag unter der Bezeichnung Epikote DX-255 erhältliches Epoxiharz mit einem Epoxiäquivalentgewicht (EEW) von 182 bis 212 und dem spezifischen Gewicht von 1,05 bei 20 °C eingesetzt wird.

9. Verfahren nach den Ansprüchen 7 bis 8, dadurch gekennzeichnet, dass als Härtungskomponente ($B_1$) ein viskoses Polyaminoamid in 20 bis 50 Gewichtsprozentiger wässriger Lösung, oder ein am Anmeldetag unter der Bezeichnung Casamide C A 360 erhältliches viskoses Polyaminoamid mit einem Aminwert von 130 bis 160 mg KOH/g, einer Viskosität von 300 bis 500 Poise bei 25 °C und einem Festkörpergehalt von 50 +/− 1 % eingesetzt wird.

10. Verfahren nach den Ansprüchen 7 bis 9, dadurch gekennzeichnet, dass als Polyelektrolyt (D) ein natürlicher Polyelektrolyt, z. B. Na-Alginat, oder ein chemisch modifiziertes natürliches Polymer in einer Konzentration von 5 bis 10 Gewichtsprozent eingesetzt wird.

11. Verfahren nach den Ansprüchen 7 bis 10, dadurch gekennzeichnet, dass als niedermolekularer Elektrolyt (E) ein Salz mit einem zum Polyelektrolyten (D) entgegengesetzt geladenen, mehrwertigen Ion, oder, dass bei Verwendung eines anionischen Polyelektrolyten (D) eine 0,05 bis 1 molare $CaCl_2$-Lösung eingesetzt wird.

12. Verfahren nach den Ansprüchen 7 bis 11, dadurch gekennzeichnet, dass zu der Harzkomponente ($A_2$), der Härtungskomponente ($B_1$) und der Biomasse ($A_1$) der Polelektrolyt (D) zugemischt und die Mischung durch periodisches Eindüsen in die wässrige Lösung des niedermolekularen Elektrolyten (E) aus wässriger $CaCl_2$. Lösung eingebracht wird.

13. Verfahren nach den Ansprüchen 7 bis 12, dadurch gekennzeichnet, dass die Porosität der Katalysatorperlen durch Änderung des Konzentrationsverhältnisses (D) : ($A_2$) + ($B_1$) + ($A_1$) eingestellt wird.

14. Verfahren nach den Ansprüchen 7 bis 13, dadurch gekennzeichnet, dass in Stufe 1 die Mischung ($A_2$) + ($A_1$) periodisch in die Mischung ($B_1$) eingedüst wird.

## Claims

1. A method for producing biocatalysts with high compressive strength (P/beads) and heavily loaded with enzymatically active substances (in gram of enzymatically active substance per gram biocatalyst), forming catalyst beads by polymer inclusion of biomass, characterized in that the mixtures produced by :

(A) suspending or dissolving an enzymatically active substance ($A_1$) of whole cells capable of reproduction or enzymes in an aqueous solution of polyelectrolytes in a concentration range between 0.5 and 15 % by weight,

(B) as a precipitation bath by dissolving a compound containing polyvalent ions charged oppositely to the polyelectrolytes in the mixture (A) in an concentration of the compound between 0.5 and 35 % by weight in water, and thereafter, in stage 1, the mixture (A) being converted by injection into the mixture (B) into beadlike particles of a certain grain size of about 0.5 to 5 mm, and said particles, when stirred, adhering to their surfaces within a period of 5 min to 4 h, and thereafter, in stage 2, the catalyst beads thus formed being filtered out with the entrapped enzymatic substances being washed with a physiological salt solution, and in stage 3, being subjected to careful drying by passing thereover ait at a temperature of up to 80 °C for up to 48 h, so that the catalyst beads are subjected to contraction and solidification of 4/5ths to 1/5ths compared with the condition prior to drying, and thereafter, in stage 4, the catalyst beads being introduced for rehardening into the precipitation bath (B) of stage 1, thus solidifying the beads by re-cross-linking and expansion, as compared with their original condition by re-network, and, in stage 5, subjecting the catalyst beads to a washing process and discharging them in the moist condition.

2. A method according to claim 1, characterised in that the enzymatically active substance ($A_1$) in the Solution (A) is an enzyme in a concentration up to 0.5 g/ml of the mixture (A), and in stage 1, the bead-like particles being solidified by slow, uniform stirring for between 0.25 to 5 h, and in stage 4, as a precipitation bath (B) the precipitation bath (B) from stage 1 being introduced or a precipitation bath (C) of a solution in an concentration range of 0.5 to 5 % by weight of a polyvalent electrolyte.

3. A method according to claims 1 and 2, characterized in that the polyelectrolyte in the mixture (A) is a natural polyelectrolyte, f. e. alginate or pectinate, in a molecular-weight range between 20,000 and 200,000, or a natural polymer after chemical modification, like carboxymethylcellulose.

4. A method according to claims 1 to 3, characterized in that by using anionic polyelectrolytes in the mixture (A) in the mixture (B), the ionic compound is a low-molecular-weight salt with polyvalent ions charged oppositely to the polyelectrolyte, or $CaCl_2$, $Al_2(SO_4)_3$, $Fe(NO_3)_3$ or mixtures thereof in a concentration range of 0.05 to 1 mole/l (litre).

5. A method according to claims 1 to 4, characterized in that the mixture (A) is made by suspending of the whole cells capable of reproduction in an aqueous, 2 to 10 % by weight Na-alginate solution, and, in stage 1, a 0.05 to 1 molar $CaCl_2$ solution being used as the precipitation bath (B) and, in stage 4, a 0.05 to 1 molar $Al_2(SO_4)_3$ solution being used as the precipitation bath (C), or by suspending or dissolving an isolated enzyme or an enzyme-complex in a 2 to 10 % by weight Na-alginate solution and, in stage 1 and 4, a 0.05 to 1 molar $Fe(NO_3)_3$ solution being used as a precipitation bath.

6. A method according to claims 1 to 5, characterized in that in stage 1 the mixture (A) being periodically injected into the mixture (B).

7. A method according to the generic part of claim 1, characterized in that a moist enzymatically active biomass ($A_1$) is mixed with a resin-component ($A_2$) consisting of a pratically pure multifunctional epoxy-prepolymer component in a weight ratio ($A_2$) : ($A_1$) of 0.5 : 1 to 5 : 1, and thereafter, in stage 1, an aqueous solution of a multifunctional hardening component ($B_1$) is mixed with the mass in a weight ratio ($B_1$) : (M) — wherein (M) = ($A_2$ + $B_1$) — of 0.2 : 1 to 0.8 : 1, and is distributed practically homogeneously with stirring, in order to produce poly-condensation, this system being mixed with an aqueous solution of a polyelectrolyte (D) in a weight ratio of 1 : 0.6 to 1 : 2.8, and thereafter the mixture being injected into an excess aqueous solution of a lower-molecular-weight electrolyte with polyvalent ions (E), thus producing bead-like particles of a specific grain-size of 0.5 to about 5 mm, and said particles being hardened by stirring for about 5 to 50 mins. into externally solidified particles, being subjected in stage 2, to a washing process, and thereafter, in stage 3, being dried carefully in contact with air at a temperature of up to 80 °C for about 30 h, so that the catalyst beads are subjected to contraction and solidification of 4/5 ths to 1/5ths compared with the condition prior to drying, and, in stage 4, being hardened out and, in stage 5, the polyelectrolyte being dissolved out of the bead-like particles by washing with an ionic solution concurrently with (E) having a concentration of 0.05 to 5 m/l, and the bead-like particles being separated and discharged in the moist condition.

8. A method according to claim 7, characterized in that the resin-component ($A_2$) is a low, viscous epoxy-resin emulsifiable in water, or a modified biphenol A/epichlorohydrin-epoxy compound, or an epoxy-resin obtainable on the date of the application under the name Epikote DX-255 with an equivalent epoxy weight (EEW) of 182 to 212, and a specific weight of 1.05 at 20 °C.

9. A method according to claims 7 to 8, characterized in that the hardening component ($B_1$) is a viscous polyaminoamide in a 20 to 50 % by weight aqueous solution, or a viscous polyaminoamide obtainable on the date of the application under the name Casamide CA 360 with an amino value of 130 to 160 mg of KOH/g, a viscosity of 300 to 500 poises at 25 °C, and a solids content of 50 +/− 1 %.

10. A method according to claims 7 to 9, characterized in that the poly-electrolyte (D) is a natural polyelectrolyte (f. e. Na-Alginat) or a chemically modified natural polymer in a concentration of 5 to 10 % by weight.

11. A method according to claims 7 to 10, characterized in that the lower-molecular-weight electrolyte (E) is a salt having polyvalent ions charged oppositely to the polyelectrolytes (D), or a 0.05 to 1 molar $CaCl_2$ solution, by using an anionic polyelectrolyte (D).

12. A method according to claims 7 to 11, characterized in that the polyelectrolyte (D) is mixed with the resin-component ($A_2$), the hardening component ($B_1$) and the biomass ($A_1$), the mixture being introduced, by periodic injection, into the aqueous solution of the lower-molecular-weight electrolyte consisting of an aqueous $CaCl_2$ solution.

13. A method according to claims 7 to 12, characterized in that the porosity of the catalyst beads is controlled by altering the concentration ratio (D) : ($A_2$) + ($B_1$) + ($A_1$).

14. A method according to claims 7 to 13, characterized in that in stage 1, the mixture ($A_2$) + ($A_1$) is periodically injected into the mixture ($B_1$).

## Revendications

1. Procédé de préparation de catalyseurs biologiques ayant une grande résistance à la pression (gramme force/perle) et très chargés en substances enzymatiquement actives (en grammes de substances enzymatiquement actives par gramme de catalyseur biologique) sous forme de perles de catalyseurs par inclusion de biomasse dans un polymère, caractérisé en ce qu'il consiste à préparer les mélanges en soi connus :

(A) en mettant en solution ou en suspension une substance enzymatiquement active ($A_1$) constituée de cellules entières susceptibles de se reproduire ou d'enzymes, dans une solution aqueuse à 0,5 à 15 % en poids d'un polyélectrolyte, et

(B) comme base de précipitation, en dissolvant dans l'eau un composé qui contient des ions plurivalents chargés de manière opposée au polyélectrolyte du mélange (A), jusqu'à une concentration du composé de 0,5 à 35 % en poids, à transformer ensuite au stade 1 d'une manière en soi connue, le mélange (A) par pulvérisation dans le mélange (B) en particules en forme de perles ayant une granulométrie déterminée de 0,5 à 5 mm, et à solidifier ces particules, tout en les agitant en surface pendant une durée de séjour de 5 minutes à 4 heures, ensuite en un deuxième stade, à séparer par filtration, d'une manière en soi connue, les perles de catalyseur formées avec la substance enzymatiquement active incluse et à laver par du sérum physiologique et en un troisième stade, à effectuer un séchage ménagé par envoi d'air à une température allant jusqu'à 80 °C pendant une durée de 48 heures, de manière à ce que les perles de catalyseur se rétractent relativement des 4/5 au 1/5 de l'état avant le séchage et se solidifient, ensuite, dans un quatrième stade, à introduire les perles de catalyseur pour les durcir encore, dans le même bain de précipitation (B) qu'au stade 1, de manière à ce que s'effectue, pour un gonflement en retour limité par rapport à l'état d'origine, une solidification des perles.de catalyseur par post-réticulation et re-réticulation, et au cinquième stade, à soumettre les perles de catalyseur à un processus de lavage et à les sortir à l'état humide.

2. Procédé suivant la revendication 1, caractérisé en ce que la substance enzymatiquement active (A₁) de la solution (A) est un enzyme et est présente en une concentration allant jusqu'à 0,5 gramme par ml de solution (A), en ce que les particules en forme de perles obtenues au premier stade sont solidifiées par agitation lente et uniforme pendant une durée de séjour de 0,25 à 5 heures, et en ce qu'au quatrième stade, on utilise comme bain de précipitation (B) le bain de précipitation (A) du premier stade ou un bain de précipitation (C) constitué d'une solution à 0,5 à 5 % en poids d'un électrolyte plurivalent.

3. Procédé suivant la revendication 1 ou 2, caractérisé en ce qu'il consiste à utiliser dans le mélange (A) comme polyélectrolyte, un polyélectrolyte naturel, par exemple un alginate ou un pectinate, dans la gamme de masses moléculaires allant de 20 000 à 200 000, ou un polymère naturel après modification chimique, par exemple une carboxyméthylcellulose.

4. Procédé suivant l'une des revendications 1 à 3, caractérisé en ce qu'il consiste, quand on utilise des polyélectrolytes anioniques dans le mélange (A), à mettre en œuvre, dans le mélange (B), à titre de composé ionique, des sels à bas poids moléculaire ayant des ions plurivalents de charge opposée au polyélectrolyte anionique de (A), par exemple CaCl₂, Al₂(SO₄)₃, Fe(NO₃)₃ ou leurs mélanges en des concentrations de 0,05 à 1 mole/litre.

5. Procédé suivant l'une des revendications 1 à 4, caractérisé en ce qu'il consiste à préparer le mélange (A) par mise en suspension de cellules entières susceptibles de se reproduire dans une solution aqueuse d'alginate de Na à 2 à 10 %, et à utiliser, au premier stade, comme bain de précipitation (B), une solution 0,05 à 1 molaire de CaCl₂, et, au quatrième stade, comme bain de précipitation (C), une solution 0,05 à 1 molaire d'Al₂(SO₄)₃, ou, à préparer le mélange (A) par mise en suspension ou en solution d'un enzyme isolé ou d'un complexe d'enzyme dans une solution d'alginate de Na à 2 à 10 % en poids, et à utiliser, au premier stade et au quatrième stade comme bain de précipitation, une solution 0,05 à 1 molaire de Fe(NO₃)₃.

6. Procédé suivant l'une des revendications 1 à 5, caractérisé en ce qu'il consiste, au premier stade, à pulvériser le mélange (A) périodiquement dans le mélange (B).

7. Procédé suivant le préambule de la revendication 1, caractérisé en ce qu'il consiste à mélanger une masse enzymatiquement active humide constituée de cellules entières susceptibles de se reproduire ou d'enzymes, à un constituant de résine (A₂) constitué d'un constituant époxy prépolymère plurifonctionnel pratiquement pur, en un rapport pondéral de (A₂) à (A₁) tel que de 0,5 : 1 à 5 : 1, ensuite, au premier stade, à mélanger une solution aqueuse d'un constituant de durcissement (B₁) plurifonctionnel au mélange (M) obtenu dans le rapport pondéral (B₁) : (M), (M) étant égal à (A₂) + (A₁), tel que 0,2 : 1 à 0,8 : 1, et à répartir d'une manière pratiquement homogène, par agitation pour initier la polycondensation et à mélanger ce système à une solution aqueuse d'un polyélectrolyte (D) dans le rapport pondéral de 1 : 6 à 1 : 2,8, ensuite à pulvériser le mélange dans une solution aqueuse préparée au préalable en excès d'un électrolyte à bas poids moléculaire ayant des ions plurivalents (E), avec création de particules en forme de perles d'une granulométrie déterminée de 0,5 à 5 mm, et à durcir ces particules sous agitation, en 5 à 50 minutes, en des particules solidifiées à l'extérieur, à les soumettre, au deuxième stade, à un processus de lavage, à les sécher ensuite, au troisième stade de manière à ménager, pendant 30 heures, par contact avec de l'air ayant une température allant jusqu'à 80 °C, de manière à ce que les perles de catalyseur se rétractent du 4/5 au 1/5 de l'état avant séchage et se solidifient, à les durcir, au quatrième stade, et au cinquième stade, à dissoudre jusqu'à une concentration de 0,05 à 5 m/l les polyélectrolytes constitués des particules en forme de perles, par un processus de lavage avec une solution ionique concurrente à la solution (E) et à séparer les particules en forme de perles à l'état humide et à les sortir.

8. Procédé suivant la revendication 7, caractérisé en ce qu'il consiste à utiliser, comme constituant de résine (A₂) une résine époxy faiblement visqueuse, émulsionnable dans l'eau, ou un composé époxybisphénol A-épichlorhydrine, modifié ou une résine époxy disponible au jour de la demande sous le nom d'Epikote DX-255, ayant un poids équivalent en époxy (PEE) de 182 à 212 et une densité de 1,05 à 20 °C.

9. Procédé suivant la revendication 7 ou 8, caractérisé en ce qu'il consiste à utiliser, comme constituant de durcissement (B₁) un polyaminoamide visqueux en solution aqueuse à 20 à 50 % en poids,

11

ou un polyaminoamide visqueux disponible au jour du dépôt sous le nom de Casamide C A 360 ayant un indice d'amine de 130 à 160 mg de KOH/gramme, une viscosité de 300 à 500 poises à 25 °C et une teneur en matières solides de 50 ± 1 %.

10. Procédé suivant l'une des revendications 7 à 9, caractérisé en ce qu'il consiste à utiliser, comme polyélectrolyte (D) un polyélectrolyte naturel, par exemple un alginate de Na, ou un polymère naturel modifié chimiquement en une concentration de 5 à 10 % en poids.

11. Procédé suivant l'une des revendications 7 à 10, caractérisé en ce qu'il consiste à utiliser comme électrolyte (E) à bas poids moléculaire, un sel ayant un ion plurivalent de charge opposée à celle du polyélectrolyte (D), ou, quand on utilise un polyélectrolyte anionique (D), une solution de $CaCl_2$ 0,05 à 1 molaire.

12. Procédé suivant l'une des revendications 7 à 11, caractérisé en ce qu'il consiste à ajouter le polyélectrolyte (D) au constituant de résine ($A_2$), au constituant de durcissement ($B_1$) et à la masse biologique ($A_1$), et à introduire le mélange, par pulvérisation périodique, dans la solution aqueuse de l'électrolyte à bas poids moléculaire (E) constituée d'une solution aqueuse de $CaCl_2$.

13. Procédé suivant l'une des revendications 7 à 12, caractérisé en ce qu'il consiste à régler la porosité des perles de catalyseur en modifiant le rapport de concentration (D) : ($A_2$) + ($B_1$) = ($A_1$).

14. Procédé suivant l'une des revendications 7 à 13, caractérisé en ce qu'il consiste, au premier stade, à pulvériser le mélange ($A_2$) + ($A_1$) périodiquement dans le mélange ($B_1$).

Verhalten des Durchmessers der Biokatalysator-
Perlen in mm und des Feststoffgehaltes in %
in Abhängigkeit von der Trocknungszeit in h.